# EUROPÄISCHE PATENTSCHRIFT

(11) **EP 0 752 409 B1**
(45) Veröffentlichungstag und Bekanntmachung des Hinweises auf die Patenterteilung: **17.01.2001**
(21) Anmeldenummer: 96110353.8
(22) Anmeldetag: 27.06.1996
(51) Int. Cl.: C07C 209/00, C07C 209/60

(54) **Verfahren zur Herstellung von Aminen aus Gemischen, die man bei der Spaltung von Erdölfraktionen erhält, an Zeolithen.**
Process for the preparation of amines from mixtures obtained by cleavage of petroleum fractions using zeolithes.
Procédé de préparation d'amines à partir de mélanges obtenus par clivage de coupes pétrolières à l'aide de zéolithes.

(30) Priorität: 04.07.1995 DE 19524240
(43) Veröffentlichungstag der Anmeldung: 08.01.1997
(73) Patentinhaber: BASF AKTIENGESELLSCHAFT, 67056 Ludwigshafen (DE)
(72) Erfinder: Dingerdissen, Uwe, Dr., 64342 Seeheim-Jugenheim (DE); Herrmann, Jürgen, 68307 Mannheim (DE); Eller, Karsten, Dr., 67059 Ludwigshafen (DE)

(56) Entgegenhaltungen:
- EP-A- 0 431 451
- EP-A- 0 587 424
- WO-A-93/17995
- US-A- 2 381 470
- US-A- 2 381 709
- US-A- 2 417 892
- US-A- 2 417 893
- CHEMICAL ABSTRACTS, vol. 111, no. 20, 13.November 1989 Columbus, Ohio, US; abstract no. 177765q, Seite 225; Spalte 2; XP002059147 & HU 47 536 A (TISZAI KOOLAJIPARI VALLALAT)
- DATABASE WPI Section Ch, Week 9431 Derwent Publications Ltd., London, GB; Class A60, AN 94-249692 XP002059148 & CA 2 092 964 A (TEXACO CHEM CO) , 4.Juni 1994

## Beschreibung

Die vorliegende Erfindung betrifft ein Verfahren zur Herstellung von Aminen durch Umsetzung von Gemischen, die man bei der Spaltung von Erdölfraktionen erhält, mit Ammoniak oder primären oder sekundären Aminen bei erhöhten Temperaturen und Drücken in Gegenwart von Zeolithen, Alumosilikaten, hydrothermal hergestellten Phosphaten, hochoberflächigen Oxiden, Pillared Clays oder säurebehandelten Schichtsilikaten.

Eine Übersicht über die Methoden zur Aminierung von Olefinen wird in "Functionalisation of Alkenes: Catalytic Amination of Monoolefins", J.J. Brunet et al. J.Mol.Catal., 49 (1989), Seiten 235 bis 259 gegeben.

Grundsätzlich gibt es zwei Katalysemechanismen. Das Olefin wird über ein Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Aus EP-A-587 424 ist ein Verfahren zur Herstellung von Aminen aus Olefinen bekannt, bei dem als Katalysatoron Y-Zeolithe eingesetzt werden.

Aus EP-A-431 451 ist ein Verfahren zur Herstellung von Aminen aus Olefinen bekannt, bei dem als Katalysatoren Galliumsilikat-Zeolithe eingesetzt werden.

Nachteilig an den zuvor genannten Verfahren ist die Verwendung der Olefine in reiner Form oder in vorgereinigten bzw. einfachen Gemischen.

Der vorliegenden Erfindung lag daher die Aufgabe zugrunde, diesen Nachteilen abzuhelfen.

Demgemäß wurde ein neues und verbessertes Verfahren zur Herstellung von Aminen durch Umsetzung von Gemischen, die man bei der Spaltung von Erdölfraktionen erhält, mit Ammoniak oder primären oder sekundären Aminen der allgemeinen Formel I 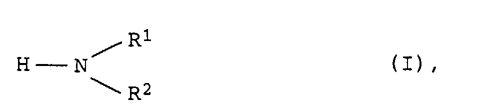 in der
- R¹,R²: Wasserstoff, C₁- bis C₈-Alkyl, C₂- bis C₈-Alkenyl, C₂H, Propargyl, C₅- bis C₈-Cycloalkyl, C₅- bis C₁₀-Alkyl-cycloalkyl, C₅- bis C₁₀-Cycloalkyl-alkyl, oder gemeinsam eine gesättigte oder ungesättigte C₃- bis C₈-Alkylendikette
bedeuten, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Zeolithe einsetzt.

Das erfindungsgemäße Verfahren kann wie folgt durchgeführt werden:

Gemische, die man bei der Spaltung von Erdölfraktionen erhält, wie beispielsweise der C4-Schnitt, der großtechnisch bei der thermischen oder katalytischen Spaltung z.B. von Naphtha anfällt, und Ammoniak oder das primäre oder sekundären Amin I können bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart eines Zeolithen als Heterogenkatalysator z.B. in einem Druck-Reaktor umgesetzt werden, und bevorzugt die erhaltenen Amine abgetrennt und die nichtumgesetzten Einsatzstoffe zurückgeführt werden.

Besonders interessant ist auch der Einsatz eines Gemisches aus C₄-Kohlenwasserstoffen, aus denen Butadien durch Extraktion oder selektive Hydrierung entfernt worden ist. Solche Gemische sind z.B. als Raffinat I in großen Mengen verfügbar. Unter geeigneten Bedingungen kann aus Raffinat I das darin enthaltende Isobuten selektiv mit Ammoniak oder Aminen zu tertiär-Butylamin bzw. anderen Aminen umgesetzt werden, ohne daß die anderen in Raffinat I enthaltenden Kohlenwasserstoffe nennenswerte Umsätze zeigen. Auf diese Weise kann die zur Herstellung von TBA bisher notwendige aufwendige Abtrennung von Isobuten aus dem Raffinat I entfallen und es ergeben sich erhebliche Vorteile ökonomischer Art.

Das vorliegende Verfahren zeichnet sich durch eine sehr gute Ausbeute bei hoher Selektivität und bei hoher Raum-Zeit-Ausbeute aus. Zudem ist keine Desaktivierung des Katalysators bemerkt worden.

Das erfindungsgemäße Verfahren zeichnet sich dadurch aus, daß bereits mit niedrigem Ammoniak- bzw. Amin-Überschuß eine hohe Selektivität an gewünschtem Reaktionsprodukt erzielt und die Dimerisierung und/oder Oligomerisierung der eingesetzten Olefine vermieden wird.

Eine Ausführungsform dieses Verfahrens besteht darin, daß man Ammoniak und/oder Amine I zusammen mit den KW-Gemischen im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt, wobei unreaktive gesättigte Kohlenwasserstoffe an dieser Stelle ohne Probleme ausgeschleust werden können.

Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen der im eingesetzten Gemisch enthaltenen Olefine gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins I und des Katalysators abhängig und liegt meist im Bereich von 200 bis 350°C.

Als Heterogenkatalysatoren für das erfindungsgemäße Verfahren eignen sich Zeolithe wie Aluminium-, Bor-, Gallium- oder Titanzeolithe des Pentasil-, Faujasit-, ZSM-12-, EMT-, SSZ-37-, CIT-1-, SSZ-33-, SSZ-26, Chinoplilolite-, Offretite-, MCM-22, PSH-3 oder BETA-Typs, bevorzugt H-ZSM-5, H-ZSM-11, H-Y, H-Aluminium-Beta, H-Bor-ZSM-5, H-Bor-ZSM-11, H-Bor-Beta, H-MCM-22, H-PSH-3 und USY.

In der Regel werden die erfindungsgemäßen Katalysatoren bevorzugt in der H-Form eingesetzt und mit einem Bindemittel im Verhältnis 98:2 bis 40:60 Gew.-% zu Strängen oder Tabletten verformt. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Presslinge zweckmäßig bei 110°C/16 h getrocknet und bei 300 bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen Katalysatoren vornehmen.

Eine Modifizierung der Katalysatoren besteht darin, daß man die unverformten oder die erfindungsgemäßen Katalysatoren Alkalimetallen wie Na und K, Erdalkalimetallen wie Ca, Mg, Erdmetallen wie Tl, Übergangsmetallen wie beispielsweise Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Katalysatoren in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen Katalysatoren vorgenommen werden.

Eine andere Möglichkeit der Metallaufbringung auf die erfindungsgemäßen Katalysatoren besteht darin, daß man das zeolithische Material z.B. mit einem Halogenid, eines Acetats, eines Oxalats, eines Citrats, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Katalysatoren kann eine Nachbehandlung mit Wasserstoff und/oder mit Wasserdampf günstig sein.

Eine weitere Möglichkeit der Modifizierung besteht darin, daß man das heterogenkatalytische Material - verformt oder unverformt - einer Behandlung mit Säuren, wie Salzsäure (HCl), Flußsäure (HF), Phosphorsäure (H₃PO₄), Schwefelsäure (H₂SO₄), Oxalsäure (HO₂C-CO₂H) oder deren Gemische unterwirft.

Eine besondere Ausführungsform besteht darin, daß man das Katalysatorpulver vor seiner Verformung mit Flußsäure 0,001 n bis 2 n, bevorzugt 0,05 bis 0,5 n 1 bis 3 Stunden unter Rückfluß behandelt. Nach Abfiltrieren und Auswaschen wird in der Regel bei 100 bis 160°C getrocknet und bei 400 bis 550°C calciniert. Eine weitere besondere Ausführungsform liegt in einer HCl-Behandlung der Heterogenkatalysatoren nach ihrer Verformung mit Bindemittel. Hierbei wird der Heterogenkatalysator in der Regel 1 bis 3 Stunden zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung ist der Austausch mit Ammonsalzen, z.B. mit NH₄Cl oder mit Mono-, Di- oder Polyaminen. Hierbei wird der mit Bindemittel verformte Heterogenkatalysator in der Regel bei 60 bis 80°C mit 10 bis 25%iger, bevorzugt 20%iger NH₄Cl-Lösung 2 h kontinuierlich in gewichtsmäßiger Heterogenkatalysator/Ammonchlorid-Lösung von 1:15 ausgetauscht und danach bei 100 bis 120°C getrocknet.

Eine weitere Modifikation, die an den erfindungsgemäßen Katalysatoren vorgenommen werden kann, ist eine Dealuminierung, bei der ein Teil der Aluminiumatome durch Silicium ersetzt wird oder die Katalysatoren durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987) Seiten 495 bis 503.

Die Katalysatoren kann man als Stränge mit Durchmessern von z.B. 1 bis 4 mm oder als Tabletten mit z.B. 3 bis 5 mm Durchmesser für die Aminierung der Kohlenwasserstoffgemische einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹ und R² in der Verbindung I haben folgende Bedeutungen:
R¹,R²
- Wasserstoff,
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₂- bis C₈-Alkenyl wie Vinyl und Allyl,
- C₂H und Propargyl,
- C₅- bis C₈-Cycloalkyl wie Cyclopentyl, Cyclohexyl, Cycloheptyl und Cyclooctyl,
- C₅- bis C₁₀-Alkyl-cycloalkyl,
- C₇- bis C₁₀-Cycloalkyl-alkyl,
- gemeinsam eine gesättigte C₃- bis C₈-Alkylendikette, bevorzugt- (CH₂)₃-, - (CH₂)₄- -(CH₂)₅-,-(CH₂)₆- und - (CH₂)₇-, insbesondere -(CH₂)₃- und -(CH₂)₄-,

- gemeinsam eine ungesättigte C₃- bis C₈-Alkylendikette, bevorzugt -CH=CH-CH=CH-.

### Beispiele

### Katalysatorverstrangung

### Katalysator A

60 g Beta-Zeolith (Fa. Uetikon) wurden mit 40 g Boehmit und 2 g Ameisensäure versetzt. Im Kneter wird die Mischung kompaktiert und unter vorsichtigem Wasserzusatz (max. 65 ml) verknetet. Die Knetzeit betrug 60 min. In einer Strangpresse wurden mit einem Preßdruck von 80 bar 2 mm Stränge erzeugt, die 16 h bei 120°C getrocknet und anschließend 16 h bei 500°C calciniert wurden.

### Katalysator B

60 g ZSM-5-Zeolith (Fa. Uetikon, PZ-2/60) wurden mit 40 g Boehmit und 2 g Ameisensäure versetzt. Im Kneter wurde die Mischung kompaktiert und unter vorsichtigem Wasserzusatz (64 ml) verknetet. Die Knetzeit betrug 40 min. In einer Strangpresse wurden mit einem Preßdruck von 55 bar 2 mm Stränge erzeugt, die 16 h bei 120°C getrocknet und anschließend 16 h bei 450°C calciniert wurden.

### Aminierungen

### Beispiele 1 bis 10

Für die kontinuierliche Herstellung wurde ein Hochdruck-Reaktor mit 2 m Länge und 24 mm Innendurchmesser eingesetzt, der mittels einer Alublockheizung beheizt und mit dreifacher Innentemperaturmessung sowie mit einer Druckhaltung ausgestattet wurde. Es wurden jeweils 60 ml Katalysator eingebaut und der obere Teil des Reaktorrohres mit Porzellanringen gefüllt. Der Zulauf von Olefin enthaltendem Gemisch aus C₄-Schnitten und Ammoniak erfolgte von oben.

Die Analyse der Reaktorausträge wurde gaschromatographisch und wahlweise zusätzlich destillativ durchgeführt.

In einen Rohrreaktor (6 mm Innendurchmesser) wurden unter isothermen Bedingungen bei 270 bis 300°C und einem Druck von 280 bar und einem Gemisch aus Ammoniak und Raffinat I [Zusammensetzung in Mol-%: 12 % Butan, 4,4 % Isobutan, 43,6 % Isobuten, 26 % Buten-1, 8,1 % trans-2-Buten und 5,9 % cis-2-Buten] und im molaren Verhältnis von 0,7:1 bis 1,3:1 NH3 zu Raffinat I umgesetzt. Die Reaktionsprodukte wurden im Gaschromatographen analysiert.

Die Ergebnisse mit den verschiedenen Katalysatoren sind in Tabelle 1 zusammengefaßt. Sie zeigen, daß es gelingt, selektiv Isobuten in einem Gemisch von C₄-Kohlenwasserstoffen mit Ammoniak zu tert.-Butylamin umzusetzen. Unter den gewählten Bedingungen werden die anderen Amine nur zu einem sehr geringen Maße zu sec.-Butylamin (2-Amino-butan) umgesetzt. Durch Veränderung von Druck und Temperatur gelingt es jedoch, Bedingungen zu finden, unter denen auch diese Reaktion abläuft.

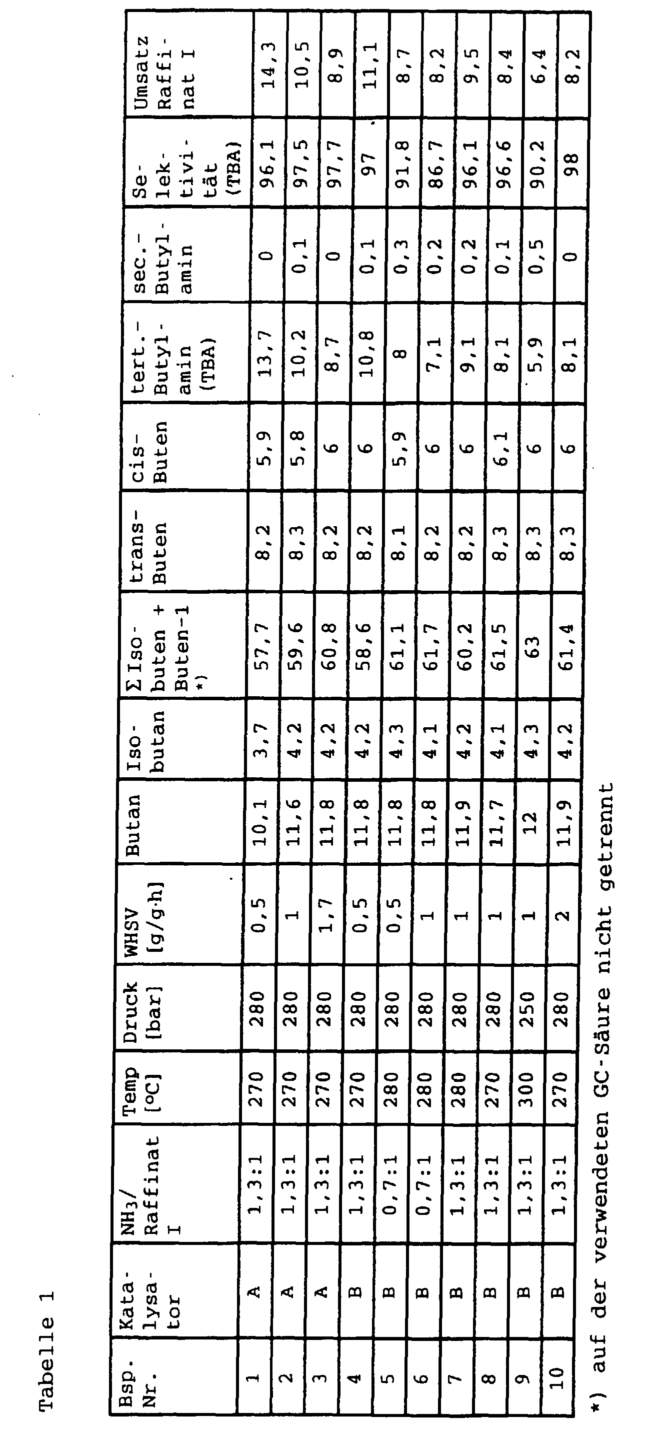

## Patentansprüche

1. Verfahren zur Herstellung von Aminen durch Umsetzung von Gemischen des C₄-Schnitts, die man bei der Spaltung von Erdölfraktionen erhält, mit Ammoniak oder primären oder sekundären Aminen der allgemeinen Formel I 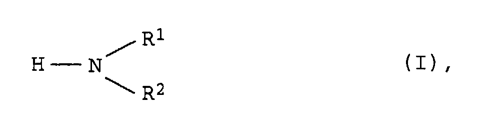 in der
R¹,R² Wasserstoff, C₁- bis C₈-Alkyl, C₂- bis C₈-Alkenyl, C₂H, Propargyl, C₅- bis C₈-Cycloalkyl, C₅- bis C₁₀-Alkyl-cycloalkyl, C₅- bis C₁₀-Cycloalkyl-alkyl oder gemeinsam eine gesättigte oder ungesättigte C₃- bis C₈-Alkylendikette
bedeuten, bei Temperaturen von 200 bis 350°C und Drücken von 100 bis 300 bar in Gegenwart eines Heterogenkatalysators, dadurch gekennzeichnet, daß man als Heterogenkatalysator Zeolithe einsetzt.

2. Verfahren zur Herstellung von Aminen I nach Anspruch 1, dadurch gekennzeichnet, daß man als Zeolithe Aluminium-, Bor-, Gallium- oder Titanzeolithe des Pentasil-, Faujasit-, EMT- oder BETA-Typs einsetzt.

3. Verfahren zur Herstellung von Aminen I nach den Ansprüchen 1 bis 2, dadurch gekennzeichnet, daß die Gemischen, die man bei der Spaltung von Erdölfraktionen erhält, Isobuten, cis-2-Buten, trans-2-Buten, 1-Buten, Isobutan und n-Butan enthalten.

4. Verfahren zur Herstellung von Aminen I nach den Ansprüchen 1 bis 3, dadurch gekennzeichnet, daß man Zeolithe in der H-Form einsetzt.

5. Verfahren zur Herstellung von Aminen nach den Ansprüchen 1 bis 4, dadurch gekennzeichnet, daß man Zeolithe einsetzt, die mit einer Säure aus der Gruppe Salzsäure, Flußsäure, Phosphorsäure, Schwefelsäure, Oxalsäure oder deren Gemischen behandelt sind.

6. Verfahren zur Herstellung von Aminen nach den Ansprüchen 1 bis 5, dadurch gekennzeichnet, daß man Zeolithe einsetzt, die mit einem oder mehreren Übergangsmetallen dotiert sind.

7. Verfahren zur Herstellung von Aminen nach den Ansprüchen 1 bis 6, dadurch gekennzeichnet, daß man Zeolithe einsetzt, die mit einem oder mehreren Elementen der Seltenen Erden oder mit einem oder mehreren Übergangsmetallen dotiert sind.

8. Verfahren zur Herstellung von Aminen nach den Ansprüchen 1 bis 7, dadurch gekennzeichnet, daß man Zeolithe einsetzt, die mit einem oder mehreren Elementen aus der Gruppe der Alkali-, Erdalkali- oder Erdmetalle dotiert sind.

9. Verfahren zur Herstellung von Aminen nach den Ansprüchen 1 bis 8, dadurch gekennzeichnet, daß man Zeolithe in der Ammoniumform einsetzt.

10. Verfahren zur Herstellung von Aminen nach den Ansprüchen 1 bis 9, dadurch gekennzeichnet, daß man Zeolithe einsetzt, die mit einem Bindemittel verformt und bei Temperaturen von 300 bis 600°C calciniert sind.

## Claims

1. A process for the preparation of amines by reacting a mixture of the C₄ fraction obtained in the cracking of mineral oil fractions with ammonia or a primary or secondary amine of the formula I 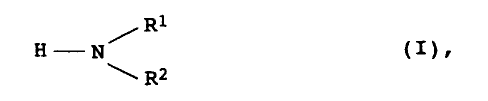 where
R¹ and R² are each hydrogen, C₁-C₈-alkyl, C₂-C₈-alkenyl, C₂H, propargyl, C₅-C₈-cycloalkyl, C₅-C₁₀-alkylcycloalkyl, C₅-C₁₀-cycloalkylalkyl or together are a saturated or unsaturated C₃-C₈-alkylene chain,
at from 200 to 350°C and from 100 to 300 bar in the presence of a heterogeneous catalyst, which comprises using zeolites as the heterogeneous catalyst:

2. A process for the preparation of amines I as claimed in claim 1, wherein an aluminum, boron, gallium or titanium zeolite of the pentasil, faujasite, EMT or BETA type is used as the zeolite.

3. A process for the preparation of amines I as claimed in claim 1 or 2, wherein the mixture obtained in the cracking of mineral oil fractions contains isobutene, cis-2-butene, trans-2-butene, 1-butene, isobutane and n-butane.

4. A process for the preparation of amines I as claimed in any of claims 1 to 3, wherein the zeolite is used in the H form.

5. A process for the preparation of amines as claimed in any of claims 1 to 4, wherein a zeolite treated with an acid selected from the group consisting of hydrochloric acid, hydrofluoric acid, phosphoric acid, sulfuric acid, oxalic acid or mixtures thereof is used.

6. A process for the preparation of amines as claimed in any of claims 1 to 5, wherein a zeolite doped with one or more transition metals is used.

7. A process for the preparation of amines as claimed in any of claims 1 to 6, wherein a zeolite doped with one or more rare earth elements or with one or more transition metals is used.

8. A process for the preparation of amines as claimed in any of claims 1 to 7, wherein a zeolite doped with one or more elements from the group consisting of alkali metals, alkaline earth metals or earth metals is used.

9. A process for the preparation of amines as claimed in any of claims 1 to 8, wherein the zeolite is used in the ammonium form.

10. A process for the preparation of amines as claimed in any of claims 1 to 9, wherein a zeolite which is molded with a binder and calcined at from 300 to 600°C is used.

## Revendications

1. Procédé de préparation d'amines par réaction de mélanges de fractions en C₄, obtenues par craquage de fractions pétrolières, avec de l'ammoniac ou des amines primaires ou secondaires de la formule générale I 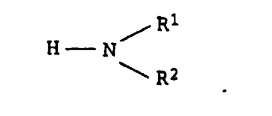 dans laquelle
R¹, R² représentent l'hydrogène, des radicaux alkyle en C₁ à C₈, alcényle en C₂ à C₈, C₂H, propargyle, cycloalkyle en C₅ à C₈, alkylcycloalkyle en C₅ à C₁₀, cycloalkylalkyle en C₅ à C₁₀, ou forment ensemble une chaîne alkylénique double saturée ou insaturée en C₃ à C₈,
à des températures de 200 à 350°C et des pressions de 100 à 300 bar, en présence d'un catalyseur hétérogène, caractérisé en ce que l'on utilise des zéolithes comme catalyseurs hétérogènes.

2. Procédé de préparation d'amines I selon la revendication 1, caractérisé en ce que l'on met en oeuvre, comme zéolithes, des zéolithes d'aluminium, de bore, de gallium ou de titane du type pentasile, faujasite, EMT ou BETA.

3. Procédé de préparation d'amines I selon les revendications 1 et 2, caractérisé en ce que les mélanges que l'on obtient par craquage de fractions pétrolières contiennent de l'isobutène, du cis-2-butène, du trans-2-butène, du 1-butène, de l'isobutane et du n-butane.

4. Procédé de préparation d'amines I selon les revendications 1 à 3, caractérisé en ce que les zéolithes sont mises en oeuvre sous leur forme H.

5. Procédé de préparation d'amines I selon les revendications 1 à 4, caractérisé en ce que l'on met en oeuvre des zéolithes traitées par un acide choisi dans le groupe formé par l'acide chlorhydrique, l'acide fluorhydrique, l'acide phosphorique, l'acide sulfurique, l'acide oxalique ou leurs mélanges.

6. Procédé de préparation d'amines I selon les revendications 1 à 5, caractérisé en ce que l'on met en oeuvre des zéolithes dopées par un ou plusieurs métaux de transition.

7. Procédé de préparation d'amines I selon les revendications 1 à 6, caractérisé en ce que l'on met en oeuvre des zéolithes dopées par un ou plusieurs éléments des terres rares ou par un ou plusieurs métaux de transition.

8. Procédé de préparation d'amines I selon les revendications 1 à 7, caractérisé en ce que l'on met en oeuvre des zéolithes dopées par un ou plusieurs éléments du groupe formé par les métaux alcalins, alcalino-terreux ou terreux.

9. Procédé de préparation d'amines I selon les revendications 1 à 8, caractérisé en ce que l'on met en oeuvre les zéolithes sous leur forme ammoniacale.

10. Procédé de préparation d'amines I selon les revendications 1 à 9, caractérisé en ce que l'on met en oeuvre des zéolithes façonnées à l'aide d'un liant et calcinées à des températures de 300 à 600°C.
